# FASCICULE DE BREVET EUROPEEN

(11) **EP 1 105 071 B1**
(45) Date de publication et mention de la délivrance du brevet: **03.03.2004**
(21) Numéro de dépôt: 99936739.4
(22) Date de dépôt: 16.08.1999
(51) Int. Cl.: A61F 2/46, A61F 2/34

(54) **COTYLE D'ESSAI OU IMPLANTABLE A ORIENTATION REGLABLE**
ORIENTIERBARE PROBE- ODER IMPLANTIERBARE GELENKPFANNE
ACETABULAR CUP FOR TESTING OR TO BE IMPLANTED WITH ADJUSTABLE ORIENTATION

(30) Priorité: 17.08.1998 FR 9810473
(43) Date de publication de la demande: 13.06.2001
(73) Titulaire: Porte, Michel, 93150 Le Blanc-Mesnil (FR)
(72) Inventeur: PORTE, Michel, F-93150 Le Blanc-Mesnil (FR); DOURSOUNIAN, Lévon, F-75116 Paris (FR)
(74) Mandataire: Jacobson, Claude
(86) Numéro de dépôt international: PCT/FR1999/001994
(87) Numéro de publication internationale: WO 2000/009045

(56) Documents cités:
- EP-A- 0 327 509
- EP-A- 0 445 068
- EP-A- 0 612 509
- EP-A- 0 630 625
- EP-A- 0 640 326
- EP-A- 0 793 949
- EP-A- 0 807 426
- WO-A-86/05384
- DE-C- 19 521 147
- US-A- 5 545 230
- US-A- 5 645 607

## Description

La présente invention concerne les prothèses totales de hanche et se rapporte plus particulièrement aux prothèses nécessitant la mise en place d'un dispositif destiné à reconstituer une cavité cotyloïdienne de l'os iliaque ayant subi des dommages dus notamment à l'arthrose, en vue de pouvoir recevoir la tête d'une prothèse fémorale.

Pour reconstituer une cavité cotyloïdienne, il est habituel de mettre en place un cotyle prothétique que l'on fixe soit par du ciment, soit par impaction, soit à l'aide de vis appropriées.

Cependant, l'orientation exacte du cotyle à implanter dans la cavité cotyloïdienne en fonction de l'orientation de l'articulation à reconstituer est très délicate dans la mesure où les repères anatomiques ne sont pas fiables.

Il s'ensuit des risques d'erreur d'orientation du cotyle, qui peuvent entraîner une mauvaise orientation de l'articulation prothétique avec des risques de luxation de la prothèse.

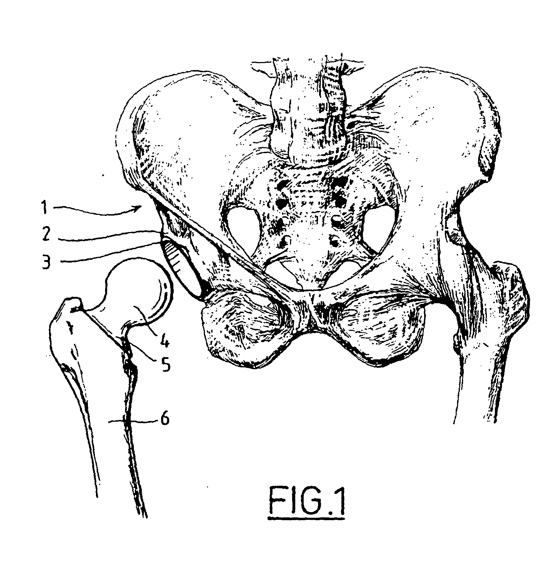

Le document EP-A2-0 807 426 montre un cotyle d'essai comprenant un insert hémisphérique (15, fig.1), une cupule hémisphérique (11, fig.1) et une embase extérieure (40, fig.1). Des moyens de liaisons (20,21 fig.1) et des moyens de réglage en position angulaire (12,14) permettent la rotation de l'insert (15) à l'intérieur de la cupule (11) autour de l'axe de l'ergot (pin (20)) alors que la cupule reste fixe dans l'embase extérieure (40) (du fait des moyens (41,42, fig.1)).

Le document EP-A1-0 445 068 montre un cotyle comprenant un insert (2, fig.1) monté à l'intérieur d'une cupule (1) possèdant des plots d'ancrage 11. Les ergots (15, fig.1) coopérant avec des logements (9, fig.1) dans la cupule empêchent toute rotation de l'insert dans celle-ci.

L'invention vise à remédier aux inconvénients de la technique antérieure en créant un cotyle implantable qui permette de choisir une orientation correcte de la cavité cotyloïdienne prothétique.

Elle a donc pour objet un cotyle d'essai ou implantable comprenant une cupule hémisphérique destinée à être mise en place dans une cavité cotyloïdienne et un insert hémisphérique en matière plastique biocompatible et destiné à être engagé de façon concentrique dans ladite cupule, caractérisé en ce qu'il comporte en outre une embase extérieure en forme de calotte sphérique pourvue de plots d'ancrage dans l'os de la cavité cotyloïdienne destinée à recevoir la cupule, une coupelle intérieure destinée à être placée dans la cupule en regard de l'embase extérieure et des moyens de liaison de l'embase extérieure et de la coupelle intérieure, l'embase et la coupelle définissant des moyens de réglage en position angulaire de la cupule dans la cavité cotyloïdienne en fonction de l'orientation à donner à l'articulation tandis que les moyens de liaison de l'embase extérieure avec la coupelle intérieure comportent des moyens d'immobilisation de la cupule par rapport à l'embase extérieure après le réglage en position angulaire de ladite cupule.

Suivant d'autres caractéristiques de l'invention :
- l'embase extérieure comporte un manchon fileté de centrage, en saillie vers l'intérieur, destiné à coopérer avec une virole de centrage extérieure prévue sur la coupelle intérieure, le manchon et la virole assurant le guidage du déplacement en translation de la coupelle intérieure par rapport à l'embase extérieure, une vis de serrage destinée à être engagée dans le manchon fileté de l'embase extérieure et dont la tête tronconique coopère avec un siège de forme correspondante ménagé dans la coupelle intérieure et coaxial à ladite virole formant avec le manchon fileté de l'embase extérieure et la virole de la coupelle intérieure, lesdits moyens de liaison de l'embase avec la coupelle;
- la cupule hémisphérique comporte un orifice central traversé par les moyens de liaison de l'embase extérieure avec la coupelle intérieure avec un jeu permettant le réglage de la position angulaire de la cupule par rapport à l'embase dans toutes les directions;
- l'insert hémisphérique est un insert d'essai qui comporte un orifice central d'accès à la vis de liaison de l'embase extérieure avec la coupelle intérieure;
- l'insert est un insert hémisphérique définitif complet substituable à l'insert d'essai après la fin de l'opération d'orientation et d'immobilisation de la cupule par rapport à l'embase extérieure;
- la cupule comporte une zone extérieure d'épaisseur réduite de réception de l'embase extérieure de façon à présenter ensemble avec l'embase extérieure, une surface extérieure à peu près continue;
- la cupule comporte dans sa paroi intérieure une portion de paroi amincie de réception de la coupelle intérieure;
- l'étendue angulaire de la zone extérieure de réception de l'embase extérieure et l'étendue angulaire de la portion de paroi intérieure de réception de la coupelle intérieure sont supérieures aux étendues angulaires de l'embase et de la coupelle d'une valeur correspondant au moins à la variation à obtenir de l'orientation angulaire de la cupule par rapport à l'embase extérieure et à la coupelle intérieure;
- la tête de la vis présente une surface d'extrémité concave tandis que le sommet de l'insert définitif présente un évidement destiné à éviter que le sommet dudit insert ne bute contre la tête de la vis.

L'invention sera mieux comprise à l'aide de la description qui va suivre, donnée uniquement à titre d'exemple et faite en se référant aux dessins annexés, sur lesquels :
- la Fig.1 est une vue schématique d'une portion de squelette humain, qui est destinée à recevoir une prothèse de hanche comportant un élément de reconstitution de la cavité cotyloïdienne;
- la Fig.2 est une vue en coupe éclatée d'un cotyle implantable suivant l'invention;
- la Fig.2A est une vue en coupe à plus grande échelle de la vis de liaison entrant dans la construction du cotyle de la Fig.2;
- la Fig.3 est une vue en coupe d'un insert définitif à mettre en place dans la cupule du cotyle de la Fig.2; et
- la Fig.4 est une vue en coupe à plus grande échelle du cotyle implantable suivant l'invention à l'état assemblé.

Sur la figure 1, on a représenté les os du bassin d'un squelette humain et en particulier, l'os iliaque 1 dont une cavité cotyloïde 2 comporte un cotyle implanté 3 destiné à son tour à recevoir la tête 4 d'une prothèse fémorale 5 fixée à la diaphyse d'un fémur 6.

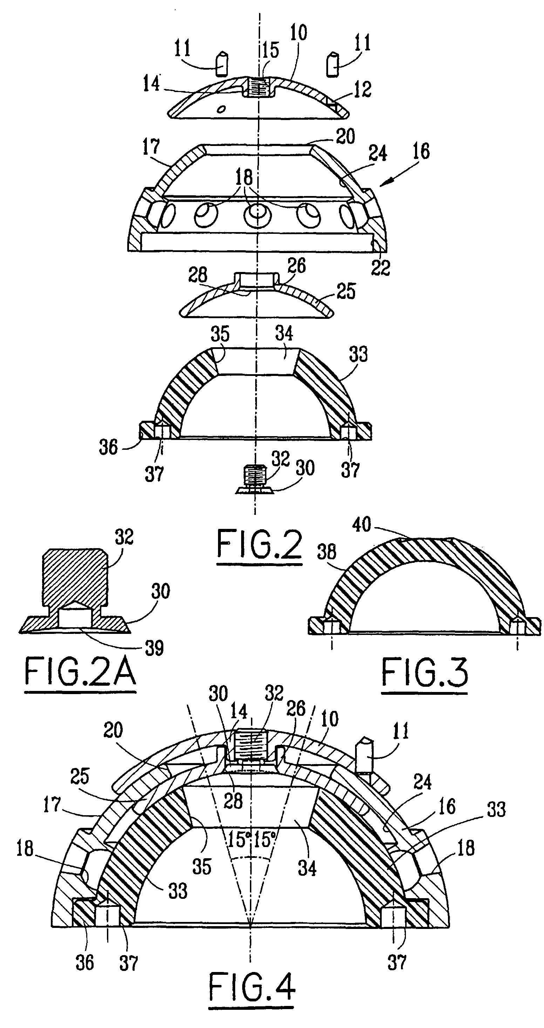

Le cotyle implantable suivant l'invention est représenté à la figure 2.

Il comporte essentiellement une embase extérieure 10 en forme de calotte sphérique et pourvue extérieurement de doigts d'ancrage 11 disposés à 120° autour de son sommet et fixés par exemple par soudage dans des empreintes 12 ménagées dans la surface extérieure de l'embase.

L'embase extérieure 10 comporte un manchon axial fileté 14 en saillie vers l'intérieur et comportant un filetage inférieur 15.

Le cotyle comporte en outre une coupelle hémisphérique 16 qui présente dans sa zone de contact avec l'embase extérieure 10, une région 17 d'épaisseur réduite destinée à absorber l'épaisseur de l'embase extérieure 10 de façon à présenter ensemble avec ladite embase extérieure, une surface extérieure à peu près continue.

On remarquera toutefois que la zone d'épaisseur réduite 17 de la cupule 16 a une étendue angulaire plus importante que celle de l'embase extérieure 10 afin de permettre le réglage angulaire de la cupule par rapport à l'embase.

La cupule 16 comporte en outre une série de trous périphériques 18 destinés à recevoir des vis (non représentées) de fixation du cotyle dans la cavité cotyloïdienne.

La cupule 16 comporte un orifice central 20 permettant son orientation angulaire par rapport à l'embase extérieure 10 sans toutefois dépasser les bords de ladite embase.

De son côté de plus grand diamètre, la cupule-16 comporte un alésage 22 destiné à recevoir le rebord de forme correspondante d'un insert qui sera décrit par la suite.

Dans la paroi intérieure de la cupule 16, est ménagée dans la zone de celle-ci, s'étendant entre les trous 18 de fixation et l'orifice central 20, une portion de paroi amincie 24 de réception d'une coupelle intérieure 25 également en forme de calotte sphérique et comportant une virole centrale 26 en saillie vers l'extérieur, destinée à coopérer avec le manchon central 14 de l'embase extérieure 10 en vue d'assurer le centrage de la coupelle intérieure 25 par rapport à l'embase extérieure 10 et leur déplacement relatif en translation d'une manière qui sera décrite par la suite.

Dans la paroi intérieure de la coupelle intérieure 25, est ménagé un siège tronconique 28 coaxial à la virole 26, de réception de la tête tronconique 30 d'une vis 32, destinée à coopérer avec le filetage intérieur 15 du manchon 14 de l'embase extérieure 10.

Le cotyle implantable comporte en outre un insert d'essai 33 en matière plastique biocompatible, réalisé par exemple en polyoxyméthylène, en polycarbonate ou en polyéthylène.

L'insert 33 est de forme hémisphérique adapté au volume intérieur de la cupule 16 et présente d'une part, un orifice central 34 à paroi tronconique 35, destiné à permettre d'accéder à la vis de liaison 32 au cours des opérations de réglage de l'orientation de la cupule 16 par rapport à l'embase extérieure 10.

Par ailleurs, l'insert 33 comporte un épaulement périphérique 36 destiné à être reçu dans l'alésage 22 de la cupule 16.

Enfin, l'insert 33 comporte deux trous de préhension 37 destinés à recevoir des ergots d'un outil préhenseur destiné à la mise en place de l'insert 33 dans la cupule 16 et à son retrait hors de cette cupule.

Comme indiqué plus haut, l'insert 33 est un insert d'essai en polyoxyméthylène résistant à de multiples stérilisations. Il peut être remplacé par un insert définitif 38 représenté à la figure 3 dont la construction est identique à celle de l'insert d'essai 33, mais dont la surface est une surface hémisphérique pleine.

L'insert définitif est réalisé en un matériau plus souple tel que du polyéthylène.

Comme on peut mieux le voir à la figure 2A, la tête 30 de la vis 32 présente une surface d'extrémité concave 39 tandis que le sommet de l'insert définitif 38 présente également un évidement 40 destiné à éviter que le sommet de l'insert définitif 38 ne bute contre la tête 30 de la vis 32.

Sur la figure 4, on a représenté le cotyle suivant l'invention en position assemblée.

L'embase extérieure 10 munie de ces doigts d'ancrage 11 est supposée être montée par impaction dans une cavité cotyloïdienne non représentée.

La cupule 16 placée contre l'embase extérieure 10 est maintenue assemblée à celle-ci par la coupelle intérieure 25 dont la virole extérieure 26 est engagée sur le manchon fileté 14 de l'embase extérieure 10.

La liaison entre l'embase extérieure 10 et la coupelle intérieure 25 est assurée par la vis 32 dont la tête tronconique 30 coopère avec le siège 28 de forme complémentaire de la coupelle intérieure 25.

Entre l'embase extérieure 10 et la coupelle intérieure 25, est ménagé un espace permettant d'assurer l'orientation angulaire de la cupule 16 par rapport à l'embase extérieure 10 dans toutes les directions.

A cet effet, le diamètre de l'orifice central 20 de la cupule 16 est telle que la cupule 16 puisse être déplacée angulairement par rapport à l'ensemble constitué par l'embase extérieure 10 et la coupelle intérieure 25 d'un angle par exemple de 15° de part et d'autre de sa position centrale représentée à la figure 3.

A cet effet, l'étendue angulaire des zones de plus faibles épaisseurs 17 et 24 réalisées dans les parois extérieure et intérieure de la cupule 16, est supérieure d'une valeur correspondante aux étendues angulaires de l'embase extérieure 10 et de la coupelle intérieure 25.

On voit donc qu'après avoir mis en place la cupule 16 entre l'embase extérieure 10 et la coupelle intérieure 25, on peut déplacer la cupule 16 angulairement dans toutes les directions entre l'embase 10 et la coupelle 25. Afin d'obtenir le réglage de l'orientation de la cupule en fonction de l'orientation à donner à l'articulation à reconstituer, on met en place dans la cupule 16, l'insert d'essai 33. Puis on introduit dans l'insert d'essai, un appareil classique de réglage de l'orientation du cotyle (non représenté) appelé porte-cotyle et on procède au réglage de l'orientation de l'articulation par déplacement de l'ensemble constitué par la cupule 16 et l'insert d'essai 33 par rapport à l'embase extérieure 10 et à la coupelle intérieure 25.

Lorsque la bonne orientation est déterminée, on retire l'appareil de réglage et on immobilise la cupule 16 par rapport à l'embase 10 en serrant la vis 32.

On introduit alors dans la cupule munie de son insert d'essai, la tête d'une prothèse fémorale d'essai, on teste les mobilités de la hanche.

Si la hanche est stable, on garde l'orientation choisie.

Si la hanche est instable, on recommence l'opération d'orientation du cotyle.

Au terme de ces opérations, l'orientation de l'articulation est déterminée de façon définitive.

Il est alors possible de retirer de la cupule l'insert d'essai 33 et de le remplacer par un insert définitif 38 de dimensions identiques à celles de l'insert d'essai 33 mais dépourvu de tout orifice central.

L'insert définitif prend dans la cupule 16, la place exacte qu'occupait l'insert d'essai 33.

Bien entendu, avant de mettre en place l'insert définitif, on fixe la cupule 16 dans la cavité cotyloïdienne à l'aide de vis non représentées engagées dans les trous périphériques 18.

Il est également possible de retirer l'ensemble et de mette en place un cotyle habituel.

Une fois cette opération effectuée, il est alors possible de mettre en place la tête de la prothèse fémorale dans le cotyle et de retrouver la bonne orientation de l'articulation ainsi reconstituée.

Les diverses pièces métalliques qui constituent le cotyle implantable suivant l'invention peuvent être réalisées en titane, en acier inoxydable, en chrome-cobalt ou en tout autre métal biocompatible.

On voit donc que grâce à l'agencement qui vient d'être décrit, il est possible d'assurer le réglage de l'orientation de la prothèse cotyloïdienne et de tester avec les pièces fémorales d'essai, la mobilité de la hanche, sans que le choix de la position du cotyle soit définitif.

## Revendications

1. Cotyle d'essai ou implantable comprenant une cupule hémisphérique (16) destinée à être mise en place dans une cavité cotyloïdienne et un insert hémisphérique (33;38) en matière plastique biocompatible et destiné à être engagé de façon concentrique dans ladite cupule, ledit cotyle comportant en outre une embase extérieure (10) en forme de calotte sphérique pourvue de plots (11) d'ancrage dans l'os de la cavité cotyloïdienne et destinée à recevoir la cupule (16), une coupelle intérieure (25) destinée à être placée dans la cupule (16) en regard de l'embase extérieure (10) et des moyens de liaison (14,26,32) de l'embase extérieure (10) et de la coupelle intérieure (25), l'embase et la coupelle définissant des moyens de réglage en position angulaire de la cupule (16) dans la cavité cotyloïdienne en fonction de l'orientation à donner à l'articulation tandis que les moyens (32) de liaison de l'embase extérieure (10) avec la coupelle intérieure (25) comportent des moyens (32) d'immobilisation de la cupule par rapport à l'embase extérieure après le réglage en position angulaire de ladite cupule.

2. Cotyle d'essai ou implantable suivant la revendication 1, **caractérisé en ce que** l'embase extérieure comporte un manchon fileté (14) de centrage, en saillie vers l'intérieur, destiné à coopérer avec une virole de centrage extérieure (26) prévue sur la coupelle intérieure (25), le manchon (14) et la virole (26) assurant le guidage du déplacement en translation de la coupelle intérieure (25) par rapport à l'embase extérieure (10), une vis de serrage (32) destinée à être engagée dans le manchon fileté (14) de l'embase extérieure (10) et dont la tête tronconique (30) coopère avec un siège (28) de forme correspondante ménagé dans la coupelle intérieure et coaxial à ladite virole (26) formant avec le manchon fileté (14) de l'embase extérieure (10) et la virole (26) de la coupelle intérieure (25), lesdits moyens de liaison de l'embase avec la coupelle.

3. Cotyle d'essai ou implantable suivant l'une des revendications 1 et 2, **caractérisé en ce que** la cupule hémisphérique comporte un orifice central traversé par la vis de l'embase extérieure (10) avec la coupelle intérieure (25) avec un jeu permettant le réglage de la position angulaire de la cupule (16) par rapport à l'embase (10) dans toutes les directions.

4. Cotyle d'essai ou implantable suivant l'une des revendications 1 à 3, **caractérisé en ce que** l'insert hémisphérique est un insert d'essai (33) qui comporte un orifice central (34) d'accès à la vis de liaison (32) de l'embase extérieure (10) avec la coupelle intérieure (25).

5. Cotyle d'essai ou implantable suivant l'une des revendications 1à 3, **caractérisé en ce que** l'insert est un insert hémisphérique définitif complet (38) substituable à l'insert d'essai (33) après la fin de l'opération d'orientation et d'immobilisation de la cupule (16) par rapport à l'embase extérieure (10).

6. Cotyle d'essai ou implantable suivant l'une des revendications 1 à 5, **caractérisé en ce que** la cupule (16) comporte une zone extérieure (17) d'épaisseur réduite de réception de l'embase extérieure (10) de façon à présenter ensemble avec l'embase extérieure (10), une surface extérieure à peu près continue.

7. Cotyle d'essai ou implantable suivant l'une des revendications 1 à 6, **caractérisé en ce que** la cupule (16) comporte dans sa paroi intérieure une portion de paroi amincie (24) de réception de la coupelle intérieure (25).

8. Cotyle d'essai ou implantable suivant l'une des revendications 6 et 7, **caractérisé en ce que** l'étendue angulaire de la zone extérieure (17) de réception de l'embase extérieure et l'étendue angulaire de la portion de paroi intérieure de réception de la coupelle intérieure sont supérieures aux étendues angulaires de l'embase et de la coupelle d'une valeur correspondant au moins à la variation à obtenir de l'orientation angulaire de la cupule (16) par rapport à l'embase extérieure (10) et à la coupelle intérieure (25).

9. Cotyle d'essai ou implantable suivant l'une des revendications 5 à 8, **caractérisé en ce que** la tête (30) de la vis (32) présente une surface d'extrémité concave (39) tandis que le sommet de l'insert définitif (38) présente un évidement (40) destiné à éviter que le sommet dudit insert (38) ne bute contre la tête (30) de la vis (32).

## Patentansprüche

1. Probe-Gelenkpfanne oder implantierbare Gelenkpfanne, umfassend eine halbkugelartige Schale (16), die dazu bestimmt ist, in einem Gelenkpfannenhohlraum angeordnet zu werden, und einen halbkugelartigen Einsatz (33; 38) aus einem biokompatiblen Kunststoff, der dazu bestimmt ist, konzentrisch in die Schale eingesetzt zu werden, wobei die Gelenkpfanne ferner einen äußeren Sockel (10) in Form einer sphärischen Kalotte umfasst, die mit Verankerungsstiften (11) zur Verankerung im Knochen des Gelenkpfannenhohlraumes versehen und dazu bestimmt ist, die Schale (16) aufzunehmen, eine innere Kappe (25) umfasst, die dazu bestimmt ist, in der Schale (16) gegenüber dem äußeren Sockel (10) angeordnet zu werden, und Mittel (14, 26, 32) zum Verbinden des äußeren Sockels (10) und der inneren Kappe (25) umfasst, wobei der Sockel und die Kappe Mittel zur Einstellung der Winkelposition der Schale (16) in dem Gelenkpfannenhohlraum in Abhängigkeit von der dem Gelenk zu gebenden Ausrichtung definieren, während die Mittel (32) zum Verbinden des äußeren Sockels (10) mit der inneren Kappe (25) Mittel (32) zum Festsetzen der Schale in Bezug auf den äußeren Sockel nach der Einstellung der Winkelposition der Schale umfassen.

2. Probe-Gelenkpfanne oder implantierbare Gelenkpfanne nach Anspruch 1,
**dadurch gekennzeichnet, dass**
der äußere Sockel eine mit einem Gewinde versehene Hülse (14) zum Zentrieren umfasst, die nach innen vorragt und dazu bestimmt ist, mit einem äußeren Zentrierring (26) zusammenzuwirken, der an der inneren Kappe (25) vorgesehen ist, wobei die Hülse (14) und der Ring (26) die Führung der Translationsverschiebung der inneren Schale (25) in Bezug auf den äußeren Sockel (10) sicherstellen, und eine Klemmschraube (32) umfasst, die dazu bestimmt ist, in die mit dem Gewinde versehene Hülse (14) des äußeren Sockels (10) einzugreifen, und deren kegelstumpfartiger Kopf (30) mit einem Sitz (28) entsprechender Form zusammenwirkt, der in der inneren Kappe vorgesehen und zu dem Ring (26) koaxial ist, wobei sie mit der mit dem Gewinde versehenen Hülse (14) des äußeren Sockels (10) und dem Ring (26) der inneren Schale (25) die Mittel zur Verbindung des Sockels mit der Kappe bildet.

3. Probe-Gelenkpfanne oder implantierbare Gelenkpfanne nach einem der Ansprüche 1 und 2,
**dadurch gekennzeichnet, dass**
die halbkugelartige Schale eine zentrale öffnung umfasst, durch die die Schraube des äußeren Sockels (10) mit der inneren Schale (25) mit einem Spiel hindurchgreift, wodurch die Einstellung der Winkelposition der Schale (16) in Bezug auf den Sockel (10) in allen Richtungen möglich ist.

4. Probe-Gelenkpfanne oder implantierbare Gelenkpfanne nach einem der Ansprüche 1 bis 3,
**dadurch gekennzeichnet, dass**
der halbkugelartige Einsatz ein Probe-Einsatz (33) ist, der eine zentralen Öffnung (34) für den Zugriff auf die Schraube (32) zum Verbinden des äußeren Sockels (10) mit der inneren Kappe (25) umfasst.

5. Probe-Gelenkpfanne oder implantierbare Gelenkpfanne nach einem der Ansprüche 1 bis 3,
**dadurch gekennzeichnet, dass**
der Einsatz ein kompletter endgültiger halbkugelartiger Einsatz (38) ist, der den Probe-Einsatz (33) nach Beendigung des Vorgangs zur Ausrichtung und Feststellung der Schale (16) in Bezug auf den äußeren Sockel (10) ersetzen kann.

6. Probe-Gelenkpfanne oder implantierbare Gelenkpfanne nach einem der Ansprüche 1 bis 5,
**dadurch gekennzeichnet, dass**
die Schale (16) eine äußere Zone (17) von geringerer Dicke zur Aufnahme des äußeren Sockels (10) umfasst, um gemeinsam mit dem äußeren Sockel (10) eine annähernd kontinuierliche Außenfläche aufzuweisen.

7. Probe-Gelenkpfanne oder implantierbare Gelenkpfanne nach einem der Ansprüche 1 bis 6,
**dadurch gekennzeichnet, dass**
die Schale (16) in ihrer Innenwand einen dünneren Wandabschnitt (24) zur Aufnahme der inneren Kappe (25) umfasst.

8. Probe-Gelenkpfanne oder implantierbare Gelenkpfanne nach einem der Ansprüche 6 und 7,
**dadurch gekennzeichnet, dass**
die Winkelausdehnung der äußeren Zone (17) zur Aufnahme des äußeren Sockels und die Winkelausdehnung des inneren Wandabschnittes zur Aufnahme der inneren Kappe um einen Wert größer als die Winkelausdehnungen des Sockels und der Kappe sind, der zumindest der zu erzielenden Veränderung der Winkelausrichtung der Schale (16) in Bezug auf den äußeren Sockel (10) und die innere Kappe (25) entspricht.

9. Probe-Gelenkpfanne oder implantierbare Gelenkpfanne nach einem der Ansprüche 5 bis 8,
**dadurch gekennzeichnet, dass**
der Kopf (30) der Schraube (32) eine konkave Endfläche (39) aufweist, während der Scheitel des endgültigen Einsatzes (38) eine Aussparung (40) aufweist, die dazu bestimmt ist zu vermeiden, dass der Scheitel des Einsatzes (38) an dem Kopf (30) der Schraube (32) anschlägt.

## Claims

1. Test or implantable acetabulum consisting of a hemispherical cup (16) intended to be inserted into a cotyloid cavity and a hemispherical insert (33;38) made of biocompatible plastic material and intended to be engaged concentrically in said cup, said acetabulum further containing an external shoulder (10) in the form of a spherical cap fitted with sockets (11) for anchoring in the bone of the cotyloid cavity and intended to receive the cup (16), an internal bowl (25) intended to be placed in the cup (16) opposite the external shoulder (10) and means of connection (14,26,32) of the external shoulder (10) and the internal bowl (25), the shoulder and the bowl defining the means of angular adjustment of the cup (16) in the cotyloid cavity as a function of the orientation to be given to the joint, while means (32) of connection of the external shoulder (10) with the internal bowl (25) consisting of means (32) of immobilisation of the cup in relation to the external shoulder after the angular adjustment of said cup.

2. Test or implantable acetabulum according to claim 1, **characterised in that** the external shoulder consists of a centring threaded sleeve tube (14) projecting towards the interior, intended to co-operate with an external centring hoop (26) which assures the guidance of the translation movement of the internal bowl (25) with respect to the external shoulder (10), a tightening screw (32) intended to engage in the threaded sleeve (14) of the external shoulder (10) and the tapered head of which (30) co-operates with a receptacle (28) of corresponding shape recessed into the internal cup and coaxial to said hoop (26), thus forming, with the threaded sleeve tube (14) of the external shoulder (10) and the hoop (26) of the internal bowl (25) said means of connection of the shoulder with the bowl.

3. Test or implantable acetabulum according to one of claims 1 and 2, **characterised in that** the hemispherical cup includes a central orifice traversed by the screw of the external shoulder (10) with the internal bowl (25) with enough play to allow the adjustment of the angular position of the cup (16) with respect to the shoulder (10) in all directions.

4. Test or implantable acetabulum according to one of claims 1 to 3, **characterised in that** the hemispherical insert is a test insert (33) which includes a central orifice (34) for access to the screw (32) connecting the external shoulder (10) with the internal bowl (25).

5. Test or implantable acetabulum according to one of claims 1 to 3, [sic] **characterised in that** the insert is a definitive, complete hemispheric insert which can be substituted for the test insert (33) after the end of the operation for orientation and immobilisation of the cup (16) with respect to the external shoulder (10).

6. Test or implantable acetabulum according to one of claims 1 to 5, **characterised in that** the cup (16) includes an exterior zone (17) of lesser thickness to receive the external shoulder (10) in such a way that together with the external shoulder (10) it presents an external surface which is more or less continuous.

7. Test or implantable acetabulum according to one of claims 1 to 6, **characterised in that** the cup (16) contains in its interior wall a thinner portion of wall (24) for reception of the internal bowl (25).

8. Test or implantable acetabulum according to one of claims 6 and 7, **characterised in that** the angular range of the exterior zone (17) for reception of the external shoulder and the angular range of the portion of interior wall for reception of the interior bowl are greater than the angular ranges of the shoulder and of the bowl by a value corresponding to at least the variation to be obtained from the angular orientation of the cup (16) in relation to the external shoulder (10) and the internal bowl (25).

9. Test or implantable acetabulum according to one of claims 5 to 8, **characterised in that** the head (30) of the screw (32) displays a surface with a concave extremity (39), while the apex of the definitive insert (38) displays a recess (40) intended to avoid the apex of said insert (38) coming into contact with the head (30) of the screw (32)
